(19) Europäisches Patentamt
European Patent Office
Office européen des brevets

(11) Veröffentlichungsnummer : **0 001 980**
**B1**

(12) # EUROPÄISCHE PATENTSCHRIFT

(45) Veröffentlichungstag der Patentschrift : **06.05.81**

(21) Anmeldenummer : **78101131.7**

(22) Anmeldetag : **13.10.78**

(51) Int. Cl.[3] : **C 07 D307/32**, C 07 C 31/34, C 07 C 67/46, C 07 C 69/72// C07C69/743

(54) **Verfahren zur Herstellung von Halogenvinyl-gamma-butyrolactonen, sowie Zwischenprodukte und ihre Herstellung.**

(30) Priorität : **26.10.77 DE 2747824**

(43) Veröffentlichungstag der Anmeldung : **30.05.79 (Patentblatt 79/11)**

(45) Bekanntmachung des Hinweises auf die Patenterteilung : **06.05.81 Patentblatt 81/18**

(84) Benannte Vertragsstaaten : **BE CH DE FR GB NL**

(56) Entgegenhaltungen :
**DE-A-2 461 525**
**DE-A-2 509 576**
**DE-A-2 702 222**
**DE-A-2 732 455**
**US-A-3 969 423**

**HOUBEN-WEYL « Methoden der organischen Chemie », 4. Auflage, Band VIII, 1952, THIEME VERLAG Stuttgart, Seiten 549 bis 550**

(73) Patentinhaber : **BAYER AG**
**Zentralbereich Patente, Marken und Lizenzen**
**D-5090 Leverkusen 1, Bayerwerk (DE)**

(72) Erfinder : **Lantzsch, Reinhard, Dr.**
**Jakob-Böhme-Strasse 12**
**D-5000 Köln (DE)**

**EP 0 001 980 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

Verfahren zur Herstellung von Halogenvinyl-gamma-butyrolactonen sowie Zwischenprodukte und ihre Herstellung

Die vorliegende Erfindung betrifft neue Verfahren zur Herstellung von teilweise bekannten Halogen-vinyl-γ-butyrolactonen, sowie Zwischenprodukte zu ihrer Herstellung.

Vinylsubstituierte γ-Butyrolactone sowie Verfahren zu ihrer Herstellung sind bereits bekannt geworden (vergleiche DT-OS 2 461 525 und 2 509 576).

Die dort beschriebenen Verfahren sind jedoch wenig wirtschaftlich und außerdem auf vinylsubstituierte bzw. dialkylvinylsubstituierte γ-Butyrolactone beschränkt.

Es ist außerdem bekannt geworden, daß 4-Methyl-3-(2', 2'-dichlorvinyl)-γ-valerolacton als Nebenprodukt bei der Verseifung von 2,2-Dimethyl-3-(2'2'-dichlorvinyl)-cyclopropan-1-carbonsäure entsteht (Vergleiche Pestic. Sci. 1974 (5) Seiten 792 bis 793).

Es ist außerdem ein Verfahren zur Herstellung von 2,2-disubstituierten Vinyl-γ-butyrolactonen bekannt geworden (vergleiche DT-OS 2 702 222), bei dem jedoch zur Herstellung der erforderlichen Ausgangsverbindung viele Stufen benötigt werden und das für eine Herstellung im technischen Maßstab zu aufwendig ist, da beispielsweise größere Mengen Aluminium- und Zinksalze anfallen.

Es wurden nun gefunden :

1. Verfahren zur Herstellung von teilweise bekannten Halogenvinyl-γ-butyrolactonen der allgemeinen Formel I

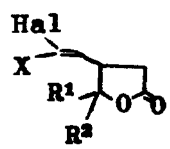

I

in welcher

Hal für F, Cl oder Br-Reste steht

X für H, F, Cl oder Br,

$R^1$ und $R^2$ für gleiche oder verschiedene Reste der Gruppe $C_{1-4}$-Alkyl stehen oder gemeinsam mit dem angrenzenden C-Atom einen cycloaliphatischen Ring mit bis zu 7 C-Atomen bilden,

dadurch gekennzeichnet, daß eine Verbindung der allgemeinen Formel II

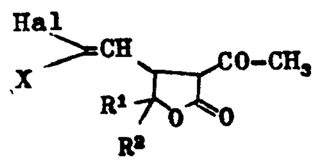

II

in welcher

Hal, X, $R^1$ und $R^2$ die oben angegebene Bedeutung haben, entacetyliert wird.

2. Neue Halogenvinyl-γ-butyrolactone der allgemeinen Formel I in welcher

Hal, X, $R^1$, $R^2$ die oben angegebene Bedeutung haben, jedoch mit der Maßgabe, daß — wenn Hal und X für Cl stehen,

$R^1$ für $C_2$-$C_4$-Alkyl steht und

$R^2$ für $C_{1-4}$-Alkyl steht sowie gemeinsam mit $R^1$ und dem angrenzenden C-Atom einen cycloaliphatischen Ring mit bis zu 7 C-Atomen bildet.

3. Neue Verbindungen der allgemeinen Formel II in welcher Hal, X, $R^1$, $R^2$ die unter 1 (oben) angegebene Bedeutung haben.

4. Verfahren zur Herstellung der Verbindungen der allgemeinen Formel II, in welcher Hal, X, $R^1$ und $R^2$ die unter 1 (oben) angegebene Bedeutung haben, dadurch gekennzeichnet, daß man aus Verbindungen der allgemeinen Formel III

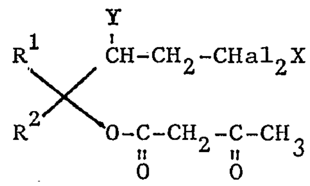

III

in welcher

Hal, X, $R^1$ und $R^2$ die unter 1 (oben) angegebene Bedeutung haben, jedoch mit der Einschränkung, daß wenn
X für H oder F steht nur einer der Reste Hal für Fluor steht und
Y für Chlor oder Brom steht,

in Gegenwart einer Base gegebenenfalls in Gegenwart eines Verdünnungsmittels zwei Mol Halogenwasserstoff abspaltet.

5. Neue Verbindungen der allgemeinen Formel III in welcher Hal, X, Y, $R^1$, $R^2$ die unter 1 (oben) angegebene Bedeutung haben.

6. Verfahren zur Herstellung der Verbindung der allgemeinen Formel III in welcher Hal, X, Y, $R^1$, $R^2$ die unter 1 (oben) angegebene Bedeutung haben, dadurch gekennzeichnet, daß man

a) Alkohole der allgemeinen Formel IV

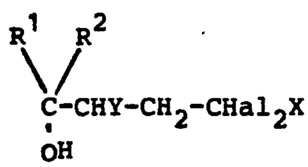

IV

in welcher

Hal, X, Y, $R^1$, $R^2$ die oben angegebene Bedeutung haben, mit Diketen gegebenenfalls in Gegenwart eines Verdünnungsmittels sowie in Gegenwart eines Katalysators umsetzt, oder

b) Verbindungen der allgemeinen Formel V

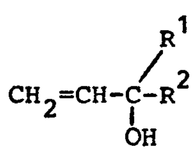

V

in welcher

$R^1$ und $R^2$ die unter 1 (oben) angegebene Bedeutung haben, mit Diketen gegebenenfalls in Gegenwart eines Verdünnungsmittels sowie in Gegenwart eines Katalysators umsetzt und anschließend die erhaltenen Verbindungen der allgemeinen Formel IX

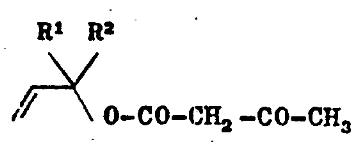

IX

in welcher

$R^1$, $R^2$ die unter 1 (oben) angegebene Bedeutung haben mit einer Verbindung der allgemeinen Formel VI

$$X\text{-}CHal_2\text{-}Y \qquad \text{VI}$$

umsetzt.

7. Neue Verbindungen der allgemeinen Formel IV, in welcher Hal, X, Y, $R^1$, $R^2$ die unter 4 (oben) angegebene Bedeutung haben.

8. Verfahren zur Herstellung der neuen Verbindungen der allgemeinen Formel IV, dadurch gekennzeichnet, daß man eine Verbindung der allgemeinen Formel V

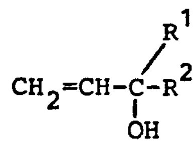

V

in welcher

$R^1$ und $R^2$ die unter 1 (oben) angegebene Bedeutung haben, mit einer Verbindung der allgemeinen Formel VI

$$X-CHal_2-Y \qquad VI$$

in welcher

X, Y und Hal die oben angegebene Bedeutung haben, mit der Maßgabe, daß nicht mehr als zwei Fluoratome im Molekül der Formel VI enthalten sein dürfen, sowie mit der Maßgabe, daß wenn X für H steht nur ein Fluoratom im Molekül der Formel IV enthalten sein darf,
umsetzt.

Das unter 1 (oben) definierte Verfahren erlaubt — ausgehend von den neuen Verbindungen II durch eine Entacetylierung, insbesondere durch eine Haloformreaktion, eine wirtschaftliche Herstellung der teilweise bekannten Halogenvinyl-γ-butyrolactone.

Verwendet man beim Verfahren 1) 2-Acetyl-4-methyl-3-(2', 2'-dichlorvinyl)-γ-valerolacton als Ausgangsstoff, kann der Reaktionsablauf durch folgendes Formelschema wiedergegeben werden :

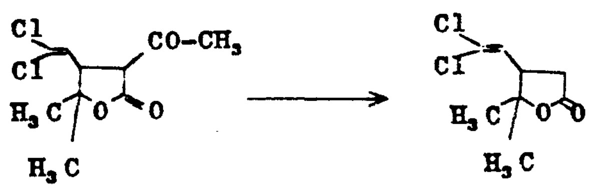

Die beim Verfahren 1) verwendbaren Ausgangsstoffe sind durch die allgemeine Formel II definiert. Darin stehen $R^1$ und $R^2$ bevorzugt für Methyl. Die Verbindungen der Formel II sind neu, ihre Herstellung wird weiter unten beschrieben.

Verfahren 1) wird durchgeführt, indem man Verbindungen der allgemeinen Formel II oxidiert. Bevorzugt wird als Oxidationsmittel Hypochlorit oder Hypobromit in wässriger Lösung verwendet, beispielsweise Natriumhypochlorit, kaliumhypochlorit, Calziumhypochlorit, Natriumhypobromit, Kaliumhypobromit und Calziumhypobromit. Die Reaktion kann auch durchgeführt werden, indem man Halogen, wie beispielsweise Chlor oder Brom in eine wässrige Alkalihydroxidlösung, wie beispielsweise Natronlauge, Kalilauge oder Kalkwasser, einbringt und mit den Verbindungen der allgemeinen Formel II zur Umsetzung bringt.

Verfahren 1) wird, gegebenenfalls in Anwesenheit von Verdünnungsmitteln, im allgemeinen bei −20 bis +100 °C durchgeführt. Als Verdünnungsmittel kommen Alkohole, wie Methanol, oder Äthanol, Äther, wie Dioxan oder Tetrahydrofuran in Betracht. Die Aufarbeitung erfolgt in der Weise, daß der Reaktionsansatz angesäuert wird, da Lactone in alkalischem Medium meist in offenkettiger Form vorliegen. Nach dem Ansäuern, beispielsweise mit Salz- oder Schwefelsäure wird gegebenenfalls zugesetztes Verdünnungsmittel abdestilliert und anschließend die Verbindungen der allgemeinen Formel I durch Abfiltrieren oder Extrahieren in üblicher Weise isoliert. Sie können — falls notwendig — durch Destillation oder Sublimation gereinigt werden.

Nach dem Verfahren 1) lassen sich bevorzugt folgende Lactone herstellen :

4-Methyl-3-(2', 2'-dichlorvinyl)-γ-valerolacton
4-Äthyl-3-(2', 2'-dichlorvinyl)-γ-valerolacton
4,4-Diäthyl-3-(2', 2'-dichlorvinyl)-γ-butyrolacton
4-Methyl-3-(2', 2'-dibromvinyl)-γ-valerolacton

4-Äthyl-3-(2', 2'-dibromvinyl)-γ-valerolacton
4,4-Diäthyl-3-(2', 2'-dibromvinyl)-γ-butyrolacton
4-Methyl-3-(2'-chlor-2'-brom)-γ-valerolacton
4-Methyl-3-(2'-fluor-2'-brom)-γ-valerolacton
4-Methyl-3-(2'-chlorvinyl)-γ-valerolacton
3-Methyl-3-(2'-bromvinyl)-γ-valerolacton

Die Verbindungen der allgemeinen Formel II sind neu. Sie werden nach dem unter 4 (oben) definierten Verfahren erhalten, indem man 1 mol eines Acetessigsäureesters der allgemeinen Formel III mit mindestens 2 Mol einer Base behandelt.

Es ist jedoch auch möglich, Verfahren 4) in zwei Stufen durchzuführen. Man erhält in diesem Falle neue Verbindungen der Formel II A

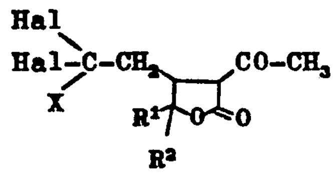

II A

in welcher

Hal, X, $R^1$ und $R^2$ die unter 4) angegebene Bedeutung haben, die dann durch weitere Behandlung mit Basen in die Verbindungen der allgemeinen Formel II überführt werden.

Verwendet man bei Verfahren 4) 2-Methyl-3,5,5,5-tetrabrompentanol-2-acetoacetat als Ausgangsstoff, läßt sich der Reaktionsablauf durch folgendes Formelschema wiedergeben :

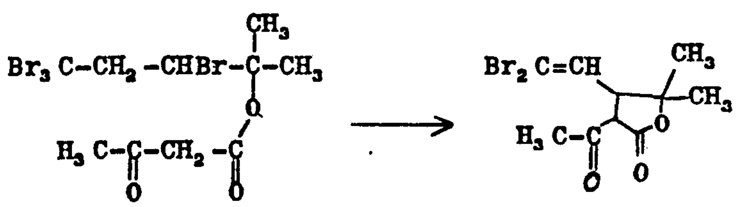

Bevorzugt werden Verbindungen der Formel III verwendet in der die Reste $R^1$, $R^2$, X, Y und Hal folgende Bedeutung haben :

$R^1$, $R^2$ steht für Methyl oder Äthyl, X steht für Wasserstoff, Fluor, Chlor oder Brom, Y steht für Chlor oder Brom, Hal steht für Fluor, Chlor oder Brom.

Besonders bevorzugt sind jedoch folgende Verbindungen der Formel III :

2-Methyl-3,5,5,5-tetrachlor-pentanol-2-acetoacetat,
2-Methyl-3,5,5,5-tetrabrom-pentanol-2-acetoacetat,
2-Methyl-5-fluor-3,5,5-trichlor-pentanol-2-acetoacetat,
2-Methyl-5-fluor-3,5,5-triborm-pentanol-2-acetoacetat,
2-Methyl-5,5-difluor-3,5-dibrom-pentanol-2-acetoacetat,
2-Methyl-3,5,5-trichlor-pentanol-2-acetoacetat,
2-Methyl-3,5,5-tribrom-pentanol-2-acetoacetat,
3-Methyl-4,6,6,6-tetrachlor-hexanol-3-acetoacetat,
3-Methyl-4,6,6,6-tetrabrom-hexanol-3-acetoacetat,
3-Methyl-6-fluor-4,6,6-trichlor-hexanol-3-acetoacetat,
3-Methyl-6,6-difluor-4,6-dibrom-hexanol-3-acetoacetat,
3-Methyl-4,6,6,-trichlor-hexanol-3-acetoacetat,
3-Methyl-4,6,6-tribrom-hexanol-3-acetoacetat.

Als Basen kommen in Frage : Alkalihydroxide, wie Natriumhydroxid, Kaliumhydroxid, Calziumhydroxid ; Alkoholate, wie Natriummethylat, Natriumäthylat oder Kalium-tert. Butylat ; ferner auch Amide wie Natriumamid.

Im allgemeinen wird in Gegenwart eines Lösungsmittels gearbeitet. Hier kommen im Falle der Alkalihydroxide vor allem Wasser oder Alkohole, wie beispielsweise Methanol, Äthanol, Glykol oder Glykolmonomethyl- oder äthyläther in Betracht.

Wird Wasser als Lösungsmittel verwendet, so wird bevorzugt im Zweiphasensystem unter Verwendung inerter organischer Lösungsmittel sowie in Gegenwart von Katalysatoren gearbeitet. Als inerte organische Lösungsmittel kommen beispielsweise Kohlenwasserstoffe, wie Benzol, Toluol, Hexan,

Cyclohexan, Chlorkohlenwasserstoffe, wie Methylenchlorid, Chlorbenzol in Frage.

Als geeignete Katalysatoren werden Verbindungen der allgemeinen Formel VII oder VIII verwendet :

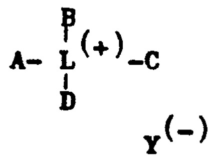

VII

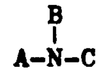

VIII

in welcher

L für Stickstoff oder Phosphor steht und die Reste

A, B, C, und D unabhängig voneinander für gegebenenfalls substituiertes Alkyl, Aralkyl oder Aryl stehen, oder zwei benachbarte Reste, A, B, C und D zusammen mit dem Zentralatom L und gegebenenfalls weiteren Heteroatomen einen Heterocyclus bilden und

Y für ein Halogenid-, Hydrogensulfat- oder Hydroxylion steht.

Die Katalysatoren der allgemeinen Formel VII sind teilweise bekannt oder können nach bekannten Verfahren hergestellt werden (Houben-Weyl, Band XI, 2 Seite 587 ff. ; Georg-Thieme-Verlag, Stuttgart 1958).

Bevorzugt sind Katalysatoren der allgemeinen Formel VII in denen

A, B, C, D für Alkyl mit 1-18 C-Atomen, insbesondere Methyl, Äthyl, Propyl, Butyl, Hexyl, Dodecyl, Octadecyl, für Benzyl, das gegebenenfalls durch $C_1$-$C_4$-Alkyl, Alkoxy oder Halogen substituiert ist, stehen.

Ferner sind bevorzugt Katalysatoren der allgemeinen Formel VIII, in denen

A, B und C für Alkyl mit 1-18-C-Atomen, insbesondere Methyl, Äthyl, Propyl, Butyl, Hexyl, Dodecyl, Octadecyl sowie für Benzyl stehen.

Als besonders bevorzugte Vertreter der beim Verfahren 4) zu verwendenden Katalysatoren seien genannt :

Tetraäthylammoniumchlorid, Tetrabutylammoniumchlorid, Tetrabutylammoniumbromid, Tetrabutylphosphoniumchlorid, Benzyltriäthylammoniumchlorid, Benzyltributylammoniumchlorid, Methyltrioctylammoniumchlorid, Triäthylamin, Tripropylamin, Tributylamin, Trihexylamin, Benzyldimethylamin.

Die Katalysatormenge kann in weiten Grenzen schwanken. Im allgemeinen haben sich 0,1 bis 10 Gew.%, vorzugsweise 0,3 bis 6 Gew.%, bezogen auf das Gewicht der eingesetzten Verbindungen der allgemeinen Formel III bewährt.

Verwendet man bei Verfahren 4) Alkoholate als Basen, werden entweder die entsprechenden Alkoholate oder aber Lösungsmittel wie Atner, z.B. Diäthyläther, Dioxan, Tetrahydrofuran oder Kohlenwasserstoffe wie Toluol verwendet.

Das erste Halogenwasserstoffmolekül läßt sich meist schon unter sehr milden Bedingungen bei etwa −20 bis +50 °C abspalten, während zur Abspaltung des zweiten Halogenwasserstoff etwas energischere Bedingungen, meist zwischen etwa +30 °C und 120 °C, notwendig sind. Bevorzugt sind daher Reaktionstemperaturen zwischen 0 und 100 °C.

Die Verbindungen der allgemeinen Formel III sind neu. Sie lassen sich nach dem unter 6 (oben) definierten Verfahren herstellen.

Verwendet man bei Verfahren 6) 2-Methyl-3,5,5,5-tetrachlorpentanol (2) und Diketen als Ausgangsstoffe, läßt sich der Reaktionsablauf durch folgendes Formelschema wiedergeben :

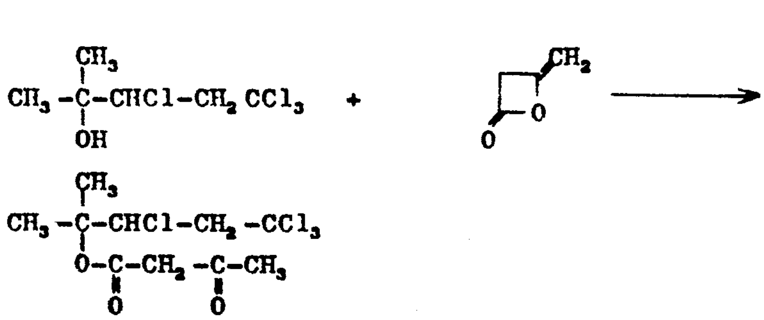

Bevorzugt werden Verbindungen der Formel IV verwendet in denen die Reste $R^1$, $R^2$, Hal, X und Y die folgende Bedeutung besitzen :

$R^1$, $R^2$ steht für Methyl oder Äthyl, Hal steht für Fluor, Chlor oder Brom, X steht für Wasserstoff, Fluor, Chlor oder Brom, Y steht für Chlor oder Brom.

Besonders bevorzugt sind jedoch folgende Verbindungen der Formel IV :

2-Methyl-3,5,5,5-tetrachlorpentanol(2) ; 2-Methyl-3,5,5,5-tetrabrom-pentanol(2) ; 2-Methyl-5-fluor-3,5,5-trichlorpentanol(2) ; 2-Methyl-5-fluor-3,5,5-tribrom-pentanol(2) ; 2-Methyl-5,5-difluor-3,5-dibrom-pentanol(2) ; 2-Methyl-3,5,5-trichlor-pentanol(2) ; 2-Methyl-3,5,5-tribrom-pentanol(2) ; 3-Methyl-4,6,6,6-tetrachlorhexanol(3) ; 3-Methyl-4,6,6,6-tetrabromhexanol(3) ; 3-Methyl-6-fluor-4,6,6-trichlorhexanol(3) ; 3-Methyl-6,6-difluor-4,6-dibrom-hexanol(3) ; 3-Methyl-4,6,6-trichlorhexanol(3) ; 3-Methyl-4,6,6-tribromhexanol(3).

Verfahren 6) wird durchgeführt, indem man 1 Mol einer Verbindung der allgemeinen Formel IV mit einem Mol Diketen gegebenenfalls in Gegenwart eines Verdünnungsmittels sowie eines Katalysators umsetzt. Vorzugsweise wird ein geringer Ueberschuß an Diketen verwendet und auf ein Verdünnungsmittel verzichtet. Arbeitet man in größerem Maßstab, kann ein solches allerdings von Vorteil sein, um die exotherme Reaktion unter Kontrolle zu halten. Als Verdünnungsmittel kommen alle organischen Lösungsmittel in Frage, die nicht mit Diketen reagieren, wie beispielsweise Kohlenwasserstoffe wie Toluol, Xylol, Cyclohexan, Halogenkohlenwasserstoffe wie Chlorbenzol, Dichlorbenzol oder Äther wie Diisopropyläther, Äthylenglykoldimethyläther.

Als geeignete Katalysatoren für Verfahren 6) seien vor allem Verbindungen der allgemeinen Formel VIII (s. oben) genannt. Die Katalysatormenge kann in weiten Grenzen schwanken. Im allgemeinen haben sich 0,1 bis 10 Gew. %, vorzugsweise 0,3 bis 6 Gew. %, bezogen auf das Gewicht der eingesetzten Verbindungen der allgemeinen Formel IV bewährt. Die Reaktionstemperatur liegt zwischen 0 °C und dem Siedepunkt des Diketens, vorzugsweise zwischen 25° und 130 °C. Es hat sich bewährt, die Stoffe der allgemeinen Formel IV und den Katalysator bei etwas erhöhter Temperatur vorzulegen und dann das Diketen zuzutropfen. Nach dem Abkühlen wird überschüssiges Diketen sowie ein gegebenenfalls zugesetztes Verdünnungsmittel abdestilliert und der Rückstand für Verfahren 4) verwendet.

Die Verbindungen der allgemeinen Formel III können auch erhalten werden, wenn man die Verbindungen der allgemeinen Formel V wie oben beschrieben mit Diketen umsetzt und die erhaltenen

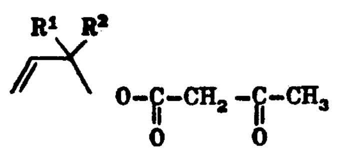 IX

mit Verbindungen der allgemeinen Formel VI umsetzt. Die Reaktion wird wie bei Verfahren 8 (siehe unten) beschrieben durchgeführt. Bevorzugt werden dabei Verbindungen der Formel eingesetzt, wobei die Reste $R^1$ und $R^2$ folgende Bedeutung haben :

$C_{1-4}$-Alkyl oder bilden gemeinsam mit dem angrenzenden C-Atom einen cycloaliphatischen Ring mit bis zu 7 C-Atomen.

Besonders bevorzugt sind folgende Verbindungen der Formel V : Isoprenalkohol (3-Methyl-1-buten-3-ol) ; 3-Methyl-1-penten-3-ol, 1-Vinyl-cyclopentan-1-ol, 1-Vinyl-cyclohexan-1-ol.

Bevorzugt werden außerdem Verbindungen der Formel VI eingesetzt, wobei die Reste X, Y und Hal folgende Bedeutung haben : X steht für Wasserstoff, Fluor, Chlor, Brom ; Y steht für Chlor, Brom ; Hal steht für Fluor, Chlor, Brom, wobei in der Verbindung der Formel VI maximal 2 Fluoratome enthalten sein sollen und für den Fall, daß X für Wasserstoff steht nur ein Fluoratom in der Verbindung der Formel VI enthalten sein soll.

Besonders bevorzugt sind folgende Verbindungen der Formel VI : $CCl_4$, $CBr_4$, $CCl_3Br$, $CF_2Br_2$, $CFBr_3$, $CClBr_3$, $CCl_3F$, $CHCl_3$, $CHBr_3$, $CHFCl_2$.

Die Verbindungen der allgemeinen Formel IV sind zum Teil bekannt. Neue und bekannte Verbindungen lassen sich nach dem unter 8 (oben) definierten Verfahren bestellen.

Verwendet man beim Verfahren 8) 3-Mehtyl-1-buten-3-ol und Tetrachlormethan als Ausgangsstoffe, läßt sich der Reaktionsablauf durch folgendes Formelschema wiedergeben :

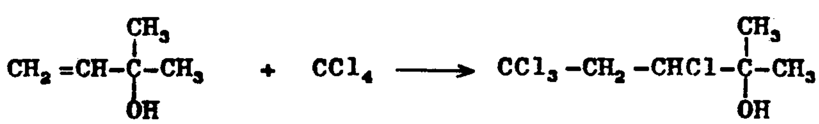

Die beim Verfahren 8) verwendbaren Ausgangsstoffe sind durch die allgemeinen Formeln V und VI definiert. Sie sind bekannt. Bevorzugt werden folgende Verbindungen der allgemeinen Formel V eingesetzt:

Isoprenalkohol (3-Methyl-1-buten-3-ol), 3-Methyl-1-penten-3-ol, 3-Äthyl-1-penten-3-ol, 1-Vinyl-cyclopentan-1-ol, 1-Vinyl-cyclohexan-1-ol.

Bevorzugt werden folgende Verbindungen der allgemeinen Formel VI eingesetzt:

$CCl_4$, $CBr_4$, $CCl_3Br$, $CF_2Br_2$, $CFBr_3$, $CClBr_3$, $CCl_3F$, $CHCl_3$, $CHBr_3$, $CHFCl_2$.

Die Umsetzung kann in Abwesenheit oder bevorzugt in Gegenwart eines Lösungsmittels durchgeführt werden. Als Lösungsmittel wird bevorzugt überschüssiges Kohlenstofftetrahalogenid verwendet, oder aber Kohlenwasserstoffe, wie Toluol, Nitrile wie Acetonitril. Besonders bevorzugt wird Acetonitril verwendet. Bevorzugt ist auch die Verwendung von Alkoholen, wie beispielsweise Propanol, Butanol oder Pentanol. Die Umsetzung wird bei Temperaturen von etwa 50-150 °C, gegebenenfalls unter Druck, durchgeführt.

Verfahren 8) kann unter Mitverwendung geeigneter Katalysatoren durchgeführt werden. Als solche kommen in Frage (für Verbindungen der allgemeinen Formel VI, in der X für H steht, kommen nur die unter 2) aufgeführten Katalysatoren in Frage):

1. Substanzen, die freie Radikale bilden, wie beispielsweise Benzoylperoxid, Acetylperoxid, Di-tert-butylperoxid, tert-Butylhydroperoxid und Azobisisobutyronitril. Im allgemeinen genügen katalytische Mengen des Radikalbildners, doch sind auch größere Mengen nicht schädlich.

2. Metallsalze in Gegenwart von organischen Aminen. Als Metallsalze kommen beispielsweise Kupfer- und Eisensalze in Betracht, wie Kupfer (I)-chlorid, Kupfer (II)-chlorid, Eissen (II) sulfat, Eisen (III)-chlorid. Als Amine kommen beispielsweise Pyrrolidin, Piperidin, n-Butylamin, Cyclohexylamin, Triäthylamin, Dimethylamin in Betracht. Sie können auch in Form ihrer Salze, beispielsweise der Hydrochloride, eingesetzt werden. Zur Umsetzung genügen ebenfalls katalytische Mengen der Katalysatoren. Größere Mengen beschleunigen die Reaktion.

3. Die Umsetzung kann auch durch Bestrahlung mit UV-Licht oder γ-Strahlen durchgeführt werden.

Verbindungen der Formel IX sind bekannt oder können nach bekannten Methoden erhalten werden (vgl. z.B. US-PS 2 795 617). Sie werden erhalten, indem man Verbindungen der Formel V mit Diketen umsetzt.

Wie bereits erwähnt, dienen die erfindungsgemäß herstellbaren Halogenvinyl-γ-butyrolactone als Zwischenprodukte zur Herstellung insektizider Wirkstoffe.

Die Herstellung dieser Wirkstoffe wird durch folgendes Formelschema illustriert:

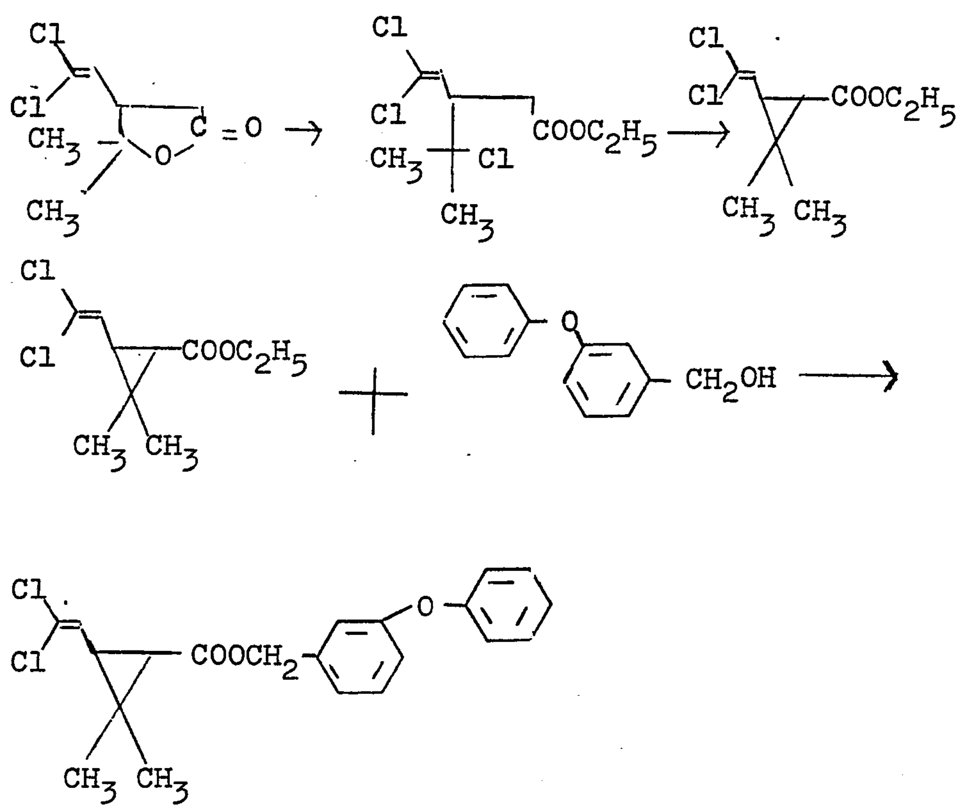

:

Der so erhaltene 2,2-Dimethyl-3(2',2'-dichlorvinyl)-cyclopropancarbonsäure-m-phenoxybenzylester

ist ein bekannter insektizider Wirkstoff (vergleiche Deutsche Offenlegungsschrift 2 326 077).

Die folgenden Beispeile erläutern das erfindungsgemäße Verfahren, ohne eine Beschränkung hinsichtlich der Breite seiner Anwendbarkeit zu geben :

Verfahren 1 : Darstellung eines γ-Butyrolactons durch Entacetylierung eines 2-Acetyl-γ-butyrolactons

Beispiel 1

25,1 g (0,1 Mol) 2-Acetyl-3-(2',2'-dichlorvinyl)-4-methyl-γ-valerolacton werden in 250 ml 10 %iger Natronlauge suspendiert und anschließend bei 0-100 °C 20 g Brom zugetropft. Nach beendetem Zutropfen wird das überschüssige Hypobromit mit wässriger Natriumsulfitlösung zerstört und die Lösung mit 25 %iger Schwefelsäure auf pH 1 gestellt. Man filtriert ab, trocknet und destilliert unter vermindertem Druck. Das 3-(2',2'-dichlorvinyl)-4-methyl-γ-valerolacton destilliert bei 160-170 °C/25 mm Hg. Die Ausbeute beträgt 17,3 g (86 % d.Th.).

Smp. 108 °C.

Beispiel 2

34 g (0,1 Mol) 2-Acetyl-3-(2',2'-dibromvinyl)-4-methyl-γ-valerolacton werden bei 0 °C in 250 ml 10 %iger Natronlauge suspendiert und anschließen bei 0-5 °C 20 g Brom zugetropft. Nach beendetem Zutropfen wird das überschüssige Hypobromit mit wäßriger Natriumbisulfitlösung zerstört und die Lösung mit 25 %iger Schwefelsäure auf pH 1 gestellt. Anschließend wird die Lösung durch Abdestillieren des Wassers konzentriert. Das 3-(2',2'-Dibromvinyl)-4-methyl-γ-valerolacton wird abfiltriert, mit Wasser gewaschen und an der Luft getrocknet.

Smp. 115-116 °C.

Beispiel 3

26,4 g (0,1 Mol) 2-Acetyl-3-(2',2'-dichlorvinyl)-4-äthyl-γ-valerolacton werden in 100 ml Dioxan gelöst. Anschließend tropft man bei 20-30 °C soviel technische Chlorlauge zu, so daß insgesamt 0,5 Mol Hypochlorit zugefügt wurden. Man erhitzt noch 3-4 h zum Sieden, zerstört evtl. noch vorhandenes Hypochlorit mit Natriumbisulfit-Lösung und stellt mit 25 %iger Schwefelsäure auf pH 1. Dann extrahiert man mit Methylenchlorid und destilliert die organische Phase fraktioniert. Man erhält 16,7 g (75 % d.Th.) 3-(2',2'-Dichlorvinyl)-4-äthyl-γ-valerolacton vom Kp = 160-165 °C/20 mmHg. Brechungsindex : $n_D^{20}$ : 1.5012.

Verfahren 4 : Darstellung eines 2-Acetyl-γ-butyrolactons durch Halogenwasserstoffabspaltung aus Acetessigestern der Formel III.

Beispiel 4

Die Lösung von 23 g Natrium in 1 000 ml Äthanol wird bei Raumtemperatur zu 162 g (0,5 Mol) Acetessigester des 2-Methyl-3,5,5,5-tetrachlorpentanol(2) getropft. Man rührt 4 h bei Raumtemperatur nach. Anschließend erhitzt man noch 4 h zum Sieden. Nach dem Abkühlen wird das gebildete Natriumchlorid abfiltriert und die Äthanollösung etwas eingeengt, mit Eiswasser versetzt und sauer gestellt. Man extrahiert mit Methylenchlorid, trocknet mit Natriumsulfat und destilliert das Lösungsmittel ab. Das entstandene 2-Acetyl-3-(2',2'-dichlorvinyl)-4-methyl-γ-valerolacton ist genügend rein, um direkt in Beispiel 1 eingesetzt zu werden. Es destilliert bei Kp 135-145 °C/0,7 mm Hg.

Beispiel 5

Analog Beispiel 4 erhält man aus dem Acetessigester des 3-Methyl-4,6,6,6-tetrachlorhexanol(3) das 2-Acetyl-3(2',2'-dichlorvinyl)-4-äthyl-γ-valerolacton.

Beispiel 6

Man tropft eine Lösung von 23 g Natrium in 1 000 ml Äthanol bei 0 °C zu 251 g (0,5 Mol) Acetessigester des 2-Methyl-3,5,5,5-tetrabrompentanol(2) und rührt 4 h bei 0 °C nach, läßt dann auf Raumtemperatur kommen und engt unter vermindertem Druck etwas ein. Die Aufarbeitung erfolgt wie in

Beispiel 4. Man erhält 125 g 2-Acetyl-3-(2',2'-dibromvinyl)-4-methyl-γ-valerolacton, das in Beispiel 2 weiterverarbeitet wird.

Beispiel 7

Zu 162 g (0,5 Mol) Acetessigester des 2-Methyl-3,5,5,5-tetrachlorpentanol(2) in 200 ml Toluol und 5 g Tetrabutylammoniumbromid werden bei Raumtemperatur 112 g 50 %ige KOH getropft. Anschließend heizt man noch 1-2 h auf 80 °C. Nach dem Abkühlen wird die wässrige Phase stark sauer gestellt und die ausgefallenen Salze mit Wasser in Lösung gebracht. Dann wird die organische Phase abgetrennt, mit Natriumsulfat getrocknet und das Lösungsmittel unter vermindertem Druck abgezogen. Man erhält 105 g 2-Acetyl-3-(2',2'-dichlorvinyl)-4-methy-γ-valerolacton.

Verfahren 6 : Umsetzung der Alkohole der allgemeinen Formel IV mit Diketen.

Beispiel 8

Zu 240 g 2-Methyl-3,5,5,5-tetrachlor-pentan-2-ol und 3 ml Triäthylamin werden bei 50 °C unter Rühren 85 g frisch destilliertes Diketen zugetropft. Hierbei steigt die Temperatur auf ca. 120 °C an. Nach dem Abkühlen wird im Wasserstrahlvakuum bei 60-70 °C andestilliert. Der Rückstand besteht aus ziemlich reinen 2-Methyl-3,5,5,5-tetrachlorpentanol(2)-acetoacetat. $n_D^{20}$ : 1.4921.

Die Struktur wird durch das Kernresonanzspektrum (in $CDCl_3$) bestätigt.

δ(ppm) = 1,6 (d, 6 Protonen, $CH_3$, $CH_3$), 2,25 (s, 3 Protonen, $CH_3$), 2,8-3,6 (m, 2 Protonen, $CH_2CCl_3$) ; 3,4 (s, 2 Protonen, $CH_2CO$) ; 4,5-4,7 (m, 1 Proton, CHCl).

Im IR-Spektrum ist keine OH-Bande mehr vorhanden.

Beispiel 9

Analog Beispiel 8 wird aus 3-Methyl-4,6,6,6-tetrachlor-hexan-3-ol und Diketen der entsprechende Acetessigester erhalten.

Verfahren 6.1 : a) Herstellung des Acetessigesters des Isoprenalkohols.

Beispiel 10

Zu 86 g Isoprenalkohol (2-Methyl-3-buten-2-ol) und 1 ml Triäthylamin werden 84 g frisch destilliertes Diketen getropft. Hierbei steigt die Temperatur auf ca. 130 °C. Nach dem Abkühlen wird der Reaktionsansatz unter vermindertem Druck destilliert. Man erhält 148 g (87 % d.Th.) 2-Methyl-3-buten-2-ol-acetoacetat vom Kp 95-100 °/15 mm Hg. $n_D^{20}$ : 1.4381.

b) Additionen von Verbindungen der allgemeinen Formel VI.

Beispiel 11

Eine Mischung von 85 g 2-Methyl-3-buten-2-ol-acetoacetat, 166 g Tetrabrommethan und 10 g Benzoylperoxid wird 3 Stunden auf 100 ° erhitzt. Nach dem Abkühlen wird in Methylenchlorid aufgenommen, mit Bicarbonatlösung und dann mit Wasser gewaschen, die organische Phase abgetrennt, mit Natriumsulfat getrocknet und das Lösungsmittel unter vermindertem Druck abdestilliert. Der zurückbleibende Acetessigester des 2-Methyl-3,5,5,5-tetrabrom-pentan-2-ol kann direkt in Beispiel 6 eingesetzt werden.

Beispiel 12

Eine Mischung von 85 g 2-Methyl-3-buten-2-ol-acetoacetat, 1 000 g Tetrachlorkohlenstoff und 20 g wasserhaltiges Benzoylperoxid wird 5 h am Wasserabscheider zum Sieden erhitzt. Nach dem Abkühlen wird mit 1 n NaOH gewaschen und anschließen das überschüssige $CCl_4$ unter vermindertem Druck abdestilliert. Der Rückstand wird im Hochvakuum destilliert. Das entstandene 2-Methyl-3,5,5,5-tetrachlor-pentan-2-ol-acetoacetat siedet bei 120-130 °C/0,4 mm Hg.

Verfahren 8 : Herstellung von Alkoholen der allgemeinen Formel IV.

Beispiel 13

430 g (5 Mol) 2-Methyl-3-buten-2-ol, 2 500 ml $CCl_4$ und 80 g wasserhaltiges Benzoylperoxid werden

10 h am Wasserabscheider zum Sieden erhitzt. Nach dem Abkühlen wird fraktioniert destilliert. Man erhält 971 g (81 % d.Th.) 2-Methyl-3,5,5,5-tetrachlorpentanol(2) vom Kp 75-85 °C/0,1 mm. $n_D^{20}$ : 1.4975.

Beispiel 14

Analog Beispiel 13 erhält man aus 3-Methyl-1-penten-3-ol in 80 %iger Ausbeute 3-Methyl-4,6,6,6-tetrachlor-hexan-3-ol vom Kp 90-98 °C/0,4 mm Hg.

Beispiel 15

In einem 0,7 1-Autoklaven werden 86 g (1 Mol) 2-Methyl-3-buten-2-ol, 420 g Difluordibrommethan und 20 g Benzoylperoxid (in Phthalat) 4 h auf 85 °C erhitzt. Nach dem Entspannen wird fraktioniert destilliert. Man erhält 285 g (96,5 % d.Th.) 2-Methyl-3,5-dibrom-5,5-difluor-pentan-2-ol vom Kp. 52 °C/0,35 mm Hg. Brechungsindex $n_D^{20}$ : 1.4747.

**Ansprüche**

1. Verfahren zur Herstellung von Halogenvinyl-γ-butyrolactonen der allgemeinen Formel I

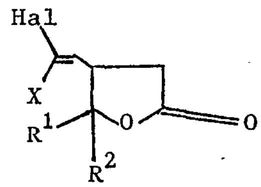

I

in welcher

Hal für F, Cl oder Br-Reste steht,
X für H, F, Cl oder Br,
$R^1$ und $R^2$ für gleiche oder verschiedene Reste der Gruppe $C_{1-4}$-Alkyl stehen oder gemeinsam mit dem angrenzenden C-Atom einen cycloaliphatischen Ring mit bis zu 7 C-Atomen bilden,

dadurch gekennzeichnet, daß eine Verbindung der allgemeinen Formel II

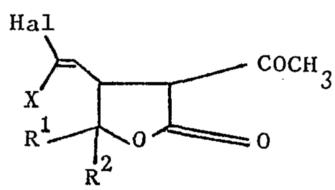

II

in welcher
Hal, X, $R^1$ und $R^2$ die oben angegebene Bedeutung haben, entacetyliert wird.

2. Verbindungen der allgemeinen Formel II in Anspruch 1, in welcher Hal, X, $R^1$ und $R^2$ die in Anspruch 1 angegebene Bedeutung haben.

3. Verfahren zur Herstellung von Verbindungen der allgemeinen Formel II, in welcher Hal, X, $R^1$ und $R^2$ die in Anspruch 1 angegebene Bedeutung haben, dadurch gekennzeichnet, daß man aus Verbindungen der allgemeinen Formel III

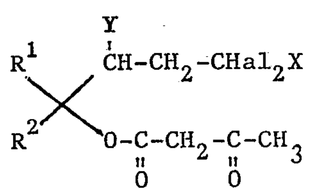

III

in welcher

Hal, X, $R^1$ und $R^2$ die in Anspruch 1 angegebene Bedeutung haben, jedoch mit der Einschränkung, daß, wenn
X für H oder F steht, nur einer der Reste Hal für Fluor steht und
Y für Chlor oder Brom steht,

in Gegenwart einer Base, gegebenenfalls in Gegenwart eines Verdünnungsmittels, zwei Mol Halogenwasserstoff abspaltet.

**Claims**

1. Process for the preparation of halogenovinyl-γ-butyrolactones of the general formula I

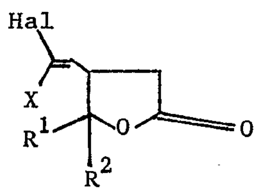

I

in which

Hal represents F, Cl or Br radicals,
X represents H, F, Cl or Br and
$R^1$ and $R^2$ represent identical or different radicals from the $C_{1-4}$-alkyl group or, together with the adjacent C atom, form a cycloaliphatic ring with up to 7 C atoms,

characterised in that a compound of the general formula II

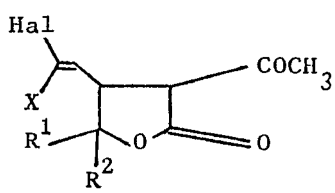

II

in which
Hal, X, $R^1$ and $R^2$ have the meaning indicated above, is deacetylated.

2. Compounds of the general formula II in Claim 1, in which Hal, X, $R^1$ and $R^2$ have the meaning indicated in Claim 1.

3. Process for the preparation of compounds of the general formula II, in which Hal, X, $R^1$ and $R^2$ have the meaning indicated in Claim 1, characterised in that two mols of hydrogen halide are split off from compounds of the general formula III

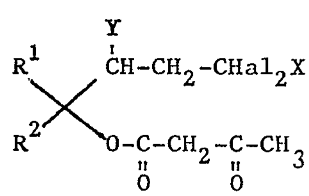

III

in which
Hal, X, $R^1$ and $R^2$ have the meaning indicated in Claim 1, but with the restriction that if X represents H or F, only one of the radicals Hal represents fluorine and Y represents chlorine or bromine,
in the presence of a base and optionally in the presence of a diluent

**Revendications**

1. Procédé de préparation d'halogénovinyl-γ-butyrolactones de formule générale I :

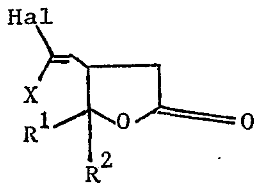

I

dans laquelle

Hal représente F, Cl ou Br,
X représente H, F, Cl ou Br,
$R^1$ et $R^2$, qui peuvent être identiques ou différents, représentent chacun un groupe alkyle contenant 1 à 4 atomes de carbone ou, ensemble avec l'atome de carbone adjacent, ils forment un noyau cycloaliphatique contenant jusqu'à 7 atomes de carbone,

caractérisé en ce qu'on soumet, à une désacétylation, un composé de formule générale II :

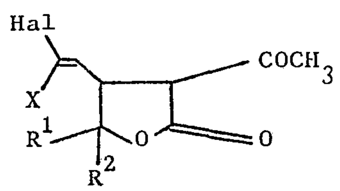

II

dans laquelle
Hal, X, $R^1$ et $R^2$ ont les significations indiquées ci-dessus.

2. Composés de formule générale II suivant la revendication 1, formule dans laquelle Hal, X, $R^1$ et $R^2$ ont les significations indiquées dans la revendication 1.

3. Procédé de préparation de composés de formule générale II dans laquelle Hal, X, $R^1$ et $R^2$ ont les significations indiquées dans la revendication 1, caractérisé en ce qu'on sépare deux moles d'un hydracide halogéné de composés de formule générale III :

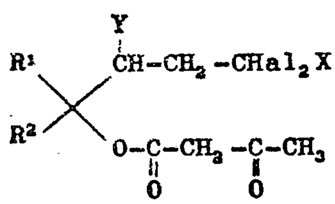

III

dans laquelle

Hal, X, $R^1$ et $R^2$ ont les significations indiquées dans la revendication 1, mais avec cette restriction que, lorsque
X représente H ou F, un seul des radicaux Hal est un atome de fluor et
Y est un atome de chlore ou de brome,

en présence d'une base et éventuellement en présence d'un diluant.